(19) Europäisches Patentamt
European Patent Office
Office européen des brevets

(11) EP 2 758 135 B1

(12) EUROPÄISCHE PATENTSCHRIFT

(45) Veröffentlichungstag und Bekanntmachung des Hinweises auf die Patenterteilung:
**19.06.2019 Patentblatt 2019/25**

(21) Anmeldenummer: **12766298.9**

(22) Anmeldetag: **17.09.2012**

(51) Int Cl.:
***A61Q 5/10*** (2006.01) ***A61K 8/36*** (2006.01)
***A61K 8/42*** (2006.01)

(86) Internationale Anmeldenummer:
**PCT/EP2012/068217**

(87) Internationale Veröffentlichungsnummer:
**WO 2013/041475 (28.03.2013 Gazette 2013/13)**

# (54) SCHAUMFÖRMIGE FÄRBEMITTEL FÜR KERATINISCHE FASERN MIT VERBESSERTEM FARBAUFZUG

FOAM DYEING AGENT FOR KERATINOUS FIBRES WITH IMPROVED COLOUR UPTAKE

COLORANTS SOUS FORME DE MOUSSE POUR FIBRES KÉRATINIQUES PERMETTANT D'OBTENIR UNE MEILLEURE APPLICATION DE LA COULEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.09.2011 DE 102011082918**

(43) Veröffentlichungstag der Anmeldung:
**30.07.2014 Patentblatt 2014/31**

(73) Patentinhaber: **Henkel AG & Co. KGaA**
**40589 Düsseldorf (DE)**

(72) Erfinder:
- **WESER, Gabriele**
**41472 Neuss (DE)**
- **SCHUMACHER, Ulrike**
**40589 Düsseldorf (DE)**

(56) Entgegenhaltungen:
**EP-A1- 1 374 837 EP-A1- 2 277 600**

- **DATABASE GNPD [Online] MINTEL; November 2008 (2008-11), "Colour Creme Gloss & Vive Pro Hydra-Gloss Shampoo Bonus Pack", XP002706285, Database accession no. 997852**
- **DATABASE GNPD [Online] MINTEL; April 2003 (2003-04), "Color Shampoo 304", XP002706286, Database accession no. 201553**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft schaumförmige Mittel und Verfahren zur oxidativen Farbveränderung keratinhaltiger Fasern, enthaltend eine spezifische Tensidkombination. Außerdem betrifft die Erfindung ein entsprechendes Mehrkomponenten-Kit zur Farbveränderung von keratinhaltigen Fasern.

Für die Farbveränderung von menschlichen Haaren kennt der Fachmann verschiedene Verfahren Zur Färbung menschlicher Haare kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Diese Kuppler- und Entwicklerkomponenten werden als Oxidationsfarbstoffvorprodukte bezeichnet. Die mit Oxidationsfarben erzielten Färbungen werden zumeist als permanente oder semipermanente Färbungen bezeichnet. Zur Aufhellung oder Blondierung werden zumeist oxidative Aufhellverfahren eingesetzt, bei denen natürliche oder synthetische Farbstoffe in der Haarfaser oxidativ zerstört und dadurch entfärbt werden.

Zumeist besitzen oxidative Färbe- und Aufhellmittel zur Reaktionsbeschleunigung während der oxidativen Anwendung einen alkalischen pH-Wert, der mit Alkalisierungsmitteln, wie Alkanolaminen, Ammoniak oder anorganischen Basen, eingestellt wird. Insbesondere Ammoniak ermöglicht dabei zwar gute Färbeergebnisse, offenbart jedoch aufgrund seines Geruchs und Reizpotentials für Haut und Schleimhäute auch Nachteile für den Anwender. Daher liegen verstärkte Bemühungen auf der Entwicklung leistungsfähiger oxidativer Färbe- und Aufhellmittel, die auf den Einsatz von Ammoniak verzichten.

Als Oxidationsmittel enthalten diese Mittel zumeist Wasserstoffperoxid. Da sich Wasserstoffperoxid im alkalischen pH-Bereich nur unzureichend lagerstabil ist, bestehen oxidative Färbe- und/oder Aufhellmittel üblicherweise aus zwei Komponenten, die unmittelbar vor der Anwendung miteinander vermischt werden. Dabei stellt sich das Problem einer homogenen Durchmischung der beiden Komponenten einerseits, was durch entsprechend leicht mischbare Komponenten, insbesondere niedrig viskose Flüssigkeiten lösbar wäre. Andererseits muss die Anwendungsmischung jedoch ausreichend ausreichend zähflüssig sein, damit sie bequem auf die Haare aufgetragen werden kann und dabei nicht tropft oder während der Einwirkzeit verläuft. Bei handelsüblichen hochviskosen Creme- oder Gel-Applikationen stellt sich häufig das Problem einer mühsamen und aufwendigen Applikation auf dem Kopfhaar, wobei eine ungleichmäßige Auftragung leicht zu inhomogenen oder gar scheckigen Färbe- oder Blondierresultaten führt. Es hat daher nicht an Versuchen gefehlt, andere Konfektionierungsformen zu entwickeln.

So wurde vorgeschlagen, dünnflüssigere Färbe- oder Aufhellmittel mit speziellen Applikatorsystemen auf die Haare aufzubringen oder Färbemittel als Schaum zu applizieren. Bei der Schaumapplikation ist Insbesondere der Einsatz von Aerosolschäumen, die durch Treibgase bei der Produktabgabe aufgeschäumt werden, verbreitet.

Zwar weist eine Schaumapplikation gegenüber Gel- oder Cremeapplikationen Vorteile in der Auftragung und Vertellung der Farbveränderungsmittel auf dem Kopfhaar des Anwenders auf. Dem stehen jedoch Nachteile in der Färbeleistung gegenüber, insbesondere im Hinblick auf den Farbaufzug, was entweder durch längere Einwirkzeiten in der Anwendung oder durch erhöhte Farbstoff-oder Aufhellmittelkonzentrationen in den Zubereitungen kompensiert werden muss. Ein weiteres Problem bei der Schaumapplikation ist die Stabilisierung der Schäume. Insbesondere die Schaumstabilität hat einen großen Einfluss sowohl auf die Leistungsfähigkeit als auch auf die leichte Anwendbarkeit der Mittel. Die Schaumstabilität wird insbesondere durch die Anwesenheit größerer Mengen an Salzen und Farbstoff(vorprodukt)en negativ beeinflusst. Die Beschaffenheit von Schäumen ist dann als ideal zu bezeichnen, wenn ein fester, stabiler Schaum vorliegt, der ein geschmeidiges Gefühl hinterlässt und auf dem Haar nur langsam bricht. Häufig ist aber zu beobachten, dass die ausgebrachten Schäume wenig stabil sind und schnell wieder in sich zusammenfallen und sich verflüssigen, so dass eine dünnflüssige, tropfende Lösung zurückbleibt. Andererseits ist es auch wesentlich, dass der Schaum trotzdem die Haare gut benetzt und langsam bricht, so dass ein guter Farbaufzug stattfinden kann.

EP2277600A1 offenbart treibmittelfreie Nonaerosol-Haarfärbemittel, die als Schaum aufgetragen werden und ein Fettsäuresalz und ein weiteres Tensid, insbesondere ein Niotensid, enthalten.

Es besteht daher weiterhin das Bedürfnis nach einer einfach zu handhabenden Darreichungsform von oxidativen Haarfarbveränderungsmittel, wie einer permanenten Haarfarbe oder Haarblondiermittel. Diese Anwendungsform soll einerseits eine tropffreie Applikation ermöglichen und andererseits in ihrer Leistungsfähigkeit den handelsüblichen Creme- oder Gel-Applikationen, insbesondere im Hinblick auf Aufhell- und Färbevermögen sowie Echtheitseigenschaften, nicht unterlegen sind. Die Färbeergebnisse sollen hervorragend im Hinblick auf Intensität, Grauabdeckung und Glanz sein, über hervorragende Pflegeeigenschaften verfügen und eine lange Haltbarkeit, auch gegenüber äußeren Einflüssen, wie Haarwäsche, aufweisen.

Aufgabe der vorliegenden Erfindung war es daher, Verfahren für oxidative Farbveränderungsmittel, insbesondere für die Schaumapplikation, so zu optimieren, dass die oben genannten Nachteile überwunden werden können. Insbesondere sollten stabile Farbveränderungsschäume bereitgestellt werden.

Es wurde überraschend gefunden, dass oxidative Farbveränderungsmittel, die eine spezifische Tensidkombination aus Seifen und nichtionischen Tensiden vom Typ der Fettsäurealkanolamide enthalten, äußerst stabile Schäume ergeben, die eine einfache und intensive Färbung der Fasern ermöglichen und zu hervorragenden Farbaufzug auf den

Fasern führen. Die Stabilität bleibt auch bei höheren Salzkonzentrationen erhalten. Diese Mittel lassen sich sehr einfach und gleichmäßig mit der Hand auf dem Haar verteilen und erlauben so eine einfache Handhabung. Außerdem ermöglichen die erfindungsgemäßen Mittel intensivere Farbergebnisse, insbesondere im Hinblick auf Intensität und erzielen deutlich haltbarere Farbergebnisse als bisher bekannte Schaumzubereitungen. Außerdem ermöglicht die Schaumapplikation einen hervorragenden Pflegeeffekt bei der Anwendung, insbesondere auch, weil eine homogene Verteilung gewährleistet wird, so dass unerwünschte hohe Lokalkonzentrationen des Färbe- oder Blondiermittels vermieden werden.

Ein erster Gegenstand der vorliegenden Erfindung ist daher eine Zubereitung zur oxidativen Farbveränderung keratinischer Fasern, in einem kosmetisch verträglichen Träger enthaltend:

(a) mindestens ein farbveränderndes Mittel und
(b) die Seife Kaliumoleat in einem Anteil von 1,5 bis 4,0 Gew.-%, bezogen auf das Gesamtgewicht der Zubereitung,
(c) zusätzlich Cocamide MEA als nichtionisches Tensid (c)

in einem Anteil von 0,6 bis 2,5 Gew.-%, bezogen auf das Gesamtgewicht der Zubereitung,
wobei Kaliumoleat und Cocamide MEA in einem Gewichtsverhältnis von 4,0 / 1 bis 1,2 / 1 enthalten sind,
dadurch gekennzeichnet, dass die Zubereitung zusätzlich mindestens ein weiteres nichtionischesTensid, ausgewählt aus Alkylpolyglucosiden und/oder alkoxylierten Fettsäureestern, enthält. Unter keratinhaltigen bzw. keratinischen Fasern sind erfindungsgemäß Wolle, Pelze, Federn und Insbesondere menschliche Haare zu verstehen. Die erfindungsgemäßen Färbe- und Aufhellverfahren können prinzipiell aber auch zur Anwendung auf anderen Naturfasern, wie z. B. Baumwolle, Jute, Sisal, Leinen, Seide oder modifizierter Naturfasern, wie beispielsweise Regeneratcellulose, Nitro-, Alkyl- oder Hydroxyalkyl- oder Acetylcellulose, verwendet werden.

Die erfindungsgemäßen Zubereitungen enthalten die Inhaltsstoffe in einem kosmetisch verträglichen Träger. Dieser kosmetische Träger ist bevorzugt wässrig, alkoholisch oder wässrig-alkoholisch. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten, sofern sie die Schaumbildung und Schaumstabilität nicht übermäßig negativ beeinflussen. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Zubereitungen das Lösungsmittel in einer Konzentration von 0,1 bis 30 Gew.-%, bevorzugt in einer Konzentration von 0,5 bis 20 Gew.-%, ganz besonders bevorzugt in einer Konzentration von 1 bis 10 Gew.-%, jeweils bezogen auf die Zubereitung, enthalten.

Als ersten Inhaltsstoff (a) enthält die Zubereitung zur Farbveränderung mindestens ein farbveränderndes Mittel. Bevorzugte farbverändernden Mittel sind dabei ausgewählt aus Oxidationsfarbstoffvorprodukten, direktziehenden Farbstoffen und Peroxosalzen.

In einer bevorzugten Ausführungsform des ersten Erfindungsgegenstands enthält die Zubereitung zur Farbveränderung als farbveränderndes Mittel mindestens ein Oxidationsfarbstoffvorprodukt. Vorzugsweise enthält die Zubereitung eine oder mehrere Entwicklerkomponenten sowie gegebenenfalls eine oder mehrere Kupplerkomponenten.

Es kann erfindungsgemäß bevorzugt sein, als Entwicklerkomponente mindestens eine Verbindung aus der Gruppe auszuwählen, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diaza-cycloheptan, 1,10-Bis-(2,5-diaminophenyl)-1,4,7,10-tetraoxaderan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,6,6-triaminopyrimidin sowie deren physiologisch verträglichen Salzen.

Die Entwicklerkomponenten werden bevorzugt in einer Menge von 0,005 bis 20 Gew.-%, vorzugsweise 0,1 bis 5 Gew.-%, insbesondere von 0,5 bis 3,5 Gew.-%, jeweils bezogen auf das Gesamtgewicht der Zubereitung, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Erfindungsgemäß bevorzugte Kupplerkomponenten werden ausgewählt aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Diaminophenoxy)ethanol, 1,3-Bis-(2,4-diaminophenoxy)propan, 1-Methoxy-2-amino-4-(2-hydroxy-ethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder deren physiologisch verträglichen Salzen.

Die Kupplerkomponenten werden bevorzugt in einer Menge von 0,005 bis 20 Gew.-%, vorzugsweise 0,1 bis 5 Gew.-%, insbesondere 0,5 bis 3 Gew.-%, jeweils bezogen auf das Gesamtgewicht der Farbveränderungszubereitung, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

In einer weiteren Ausführungsform enthält die Farbveränderungszubereitung als farbveränderndes Mittel mindestens einen direktziehenden Farbstoff. Der direktziehende Farbstoff wird entweder zur Nuancierung von Aufhellmitteln oder Oxidationsfärbungen eingesetzt oder auch als alleiniges farbveränderndes Mittel enthalten sein.

Bei direktziehenden Farbstoffen handelt es sich um Farbstoffmoleküle, die direkt auf das Substrat aufziehen und keinen oxidativen Prozess zur Ausbildung der Farbe benötigen. Geeignete direktziehende Farbstoffe sind Nitrophenylendiamine, Nitroaminophenole, Azofarbstoffe, Anthrachinone oder Indophenole. Die direktziehenden Farbstoffe werden jeweils bevorzugt in einer Menge von 0,001 bis 20 Gew.-%, insbesondere 0,02 bis 5 Gew.-% und besonders von 0,05 bis 1,0 Gew.-%, jeweils bezogen auf das Gesamtgewicht der Farbveränderungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 sowie Tetrabromphenolblau und Bromphenolblau bekannten Verbindungen. Bevorzugte kationische direktziehende Farbstoffe sind Basic Blue 7, Basic Blue 26, Basic Violet 2 und Basic Violet 14,Basic Yellow 57, Basic Red 76, Basic Blue 99, Basic Brown 16 und Basic Brown 17, Basic Yellow 87, Basic Orange 31 und Basic Red 51.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

In einer weiteren Ausführungsform enthält die Farbveränderungszubereitung als farbveränderndes Mittel mindestens ein Peroxosalz. Erfindungsgemäß geeignete Peroxosalze sind insbesondere Ammoniumpersulfat, Natriumpersulfat, Kaliumpersulfat sowie deren Mischungen.

Bevorzugt enthält das anwendungsbereite Mittel die Peroxosalze in einem Gewichtsanteil von 1,0 bis 25 Gew.-% bevorzugt 3 bis 20 Gew.-% und insbesondere von 5 bis 15 Gew.-%, jeweils bezogen auf das Gesamtgewicht der Farbveränderungszubereitung.

Als weiteren wesentlichen Inhaltsstoff enthält die Zubereitung gemäß erstem Erfindungsgegenstand Kaliumoleat.

Es zeigte sich, dass gerade Kaliumoleat sich hervorragend In die Zubereitungen einarbeiten lässt und dabei

entscheidend zur Verbesserung des Farbaufzugs beiträgt.

Zur besseren Entfaltung der Wirkung ist ein bestimmter Anteil an Kaliumoleat in den Zubereitungen günstig. Erfindungsgemäße Zubereitungen sind daher dadurch gekennzeichnet, dass Kaliumoleat, in einem Anteil von 1,5 bis 4,0 Gew.-%, bezogen auf das Gesamtgewicht der Zubereitung, enthalten ist.

Als dritten wesentlichen Inhaltsstoff enthält die Zubereitung des ersten Erfindungsgegenstands Cocamide MEA in einem Anteil von 0,6 bis 2,5 Gew.-%, bezogen auf das Gesamtgewicht der Zubereitung.

Für die Verbesserung der Schaumstabilität und des Farbaufzugs ist es vorteilhaft, das Cocamide MEA in bestimmten Mengenanteilen in die Zubereitung einzuarbeiten. Erfindungsgemäße Zubereitungen sind daher dadurch gekennzeichnet, dass Cocamide MEA in einem Anteil von 0,6 bis 2,5 Gew.-%, bezogen auf das Gesamtgewicht der Zubereitung, enthalten ist.

Ganz besonders vorteilhafte Schaumstabilitäten und Farbveränderungsergebnisse lassen sich dadurch erzielen, dass Kaliumoleat und Cocamide MEA in einem bestimmten Gewichtsverhältnis zueinander eingesetzt werden.

Erfindungsgemäße Zubereitungen enthalten Kaliumoleat und Cocamide MEA im Gewichtsverhältnis von 4,0/1 bis 1,2/1.

Die Wirkung der Zubereitungen wird durch den Zusatz weiterer nichtionischer Tenside weiter verbessert.

Die Ausführungsform des ersten Erfindungsgegenstands ist daher dadurch gekennzeichnet, dass die Zubereitung zusätzlich mindestens ein weiteres, nichtionisches Tensid, ausgewählt aus Alkylpolyglucosiden und/oder alkoxylierten Fettsäureestern, enthält.

Alkylpolyglucoside im Sinne der Erfindung sind Verbindungen der Formel (II)

$$R'\text{-}O\text{-}[G]_p \qquad (II),$$

in der R' für einen Alkyl- oder Alkenylrest mit 4 bis 22 Kohlenstoffatomen, G für einen Zuckerrest mit 5 oder 6 Kohlenstoffatomen und p für eine Zahle von 1 bis 10 steht.

Der Zuckerrest G leitet sich dabei von Aldosen oder Ketosen mit 5 oder 6 Kohlenstoffatomen, vorzugsweise von Glucose, ab.

Die Indexzahl p in der allgemeinen Formel (II) gibt den Polymerisierungsgrad (DP), d. h. die Verteilung von Mono- und Polyglucosiden an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkylpolyglucosid eine analytisch ermittelte rechnerische Größe. Vorzugsweise werden Alkylpolyglucoside mit einem mittleren Oligomerisierungsgrad p von 1,1 bis 3,0 eingesetzt. Aus anwendungstechnischer Sicht sind Alkylpolyglucoside bevorzugt, deren Polymerisierungsgrad (d.h. deren Zahl p) kleiner als 1,7 ist und insbesondere zwischen 1,2 und 1,4 liegt.

Der Alkylrest R' kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkylpolyglucoside der Kettenlänge $C_8$-$C_{10}$ (DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können sowie Alkylpolyglucoside auf Basis technischer $C_{9/11}$-Oxoalkohole (DP = 1 bis 3). Der Alkylrest R' kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkylpolyglucoside auf Basis von gehärtetem $C_{12/14}$-Kokosalkohol mit einem DP von 1 bis 3.

Bevorzugt steht R' für eine Cocosalkylgruppe, eine Stearylgruppe, eine Cetylgruppe, eine Laurylgruppe, Gemische aus $C_8$-$C_{10}$-Alkylgruppen. Gemische aus $C_{12/14}$-Alkylgruppen und/oder deren Gemische.

Erfindungsgemäß besonders geeignete Alkylpolyglucoside werden unter der INCI-Bezeichnung Coco-Glucoside und dem Handelsnamen Plantacare 818 UP oder unter der INCI-Bezeichnung Lauryl-Glucoside und dem Handelsnamen Plantacare 1200 UP vertrieben.

Es ist bevorzugt, die Alkylpolyglucoside in einem Anteil von 0,1 bis 12 Gew.-%, bevorzugt von 0,5 bis 10 Gew.-%, insbesondere bevorzugt von 1,0 bis 9 Gew.-% und weiter bevorzugt von 1,5 bis 7,5 Gew.-%, jeweils bezogen auf das Gesamtgewicht der Zubereitung, einzusetzen.

(Poly)alkoxylierte Fettsäureester im Sinne der vorliegenden Erfindung sind Verbindungen, die einen Ester einer Fettsäure beinhalten und weiterhin eine mit einer oder mehreren Alkoxygruppenveretherte Hydroxygruppe enthalten. Bevorzugt handelt es sich bei den Fettsäureestern um Fettsäuretriglyceride, deren Fettsäuren weiterhin mindestens eine polyalkoxylierte Hydroxygruppe aufweisen. Besonders bevorzugte Fettsäuretriglyceride sind dabei Rincinusöl und gehärtetes Ricinusöl. Die Polyalkoxylierung geschieht zumeist mit Ethylenoxid, der Alkoxylierungsgrad (d.h. die umgesetzte Anzahl Mol Ethylenoxid pro Mol Hydroxygruppe des Fettsäureesters) beträgt bevorzugt mindestens 20, besser

40. Beispiele für erfindungsgemäß geeignete (Poly)alkoxylierte Fettsäureester werden unter den INCI-Bezeichnungen PEG-2 Hydrogenated Castor Oil, PEG-5 Hydrogenated Castor Oil, PEG-7 Hydrogenated Castor Oil, PEG-10 Hydrogenated Castor Oil, PEG-25 Hydrogenated Castor Oil, PEG-30 Hydrogenated Castor Oil, PEG-40 Hydrogenated Castor Oil, PEG-50 Hydrogenated Castor Oil, PEG-54 Hydrogenated Castor Oil, PEG-60 Hydrogenated Castor Oil, PEG-35 Castor Oil, PEG-36 Castor Oil, PEG-40 Castor Oil, PEG-200 Castor Oil vertrieben. Erfindungsgemäß besonders geeignete (poly)alkoxylierte Fettsäureester werden unter der INCI-Bezeichnung PEG-40 Hydrogenated Castor Oil und dem Handelsnamen Cremophor CO 40 (BASF) oder unter der INCI-Bezeichnung PEG-60 Hydrogenated Castor Oil und dem Handelsnamen Cremophor CO 60 (BASF) vertrieben.

Es ist bevorzugt, die (poly)alkoxylierte Fettsäureester in einem Anteil von 0,1 bis 10 Gew.-%, bevorzugt von 0,5 bis 9 Gew.-%, insbesondere bevorzugt von 1,0 bis 7,5 Gew.-% und weiter bevorzugt von 1,5 bis 5,0 Gew.-%, jeweils bezogen auf das Gesamtgewicht der Zubereitung, einzusetzen.

Weiterhin wurde gefunden, dass in den erfindungsgemäßen Zubereitungen der Zusatz von bestimmten, hydrophil modifizierten Siliconen einen positiven Effekt auf Schaumstabilität und Farbaufzug ausübt. Bevorzugte hydrophil modifizierte Silicone sind dabei bestimmte polyalkoxylierte Silicone.

Eine weitere Ausführungsform des ersten Erfindungsgegenstands ist daher dadurch gekennzeichnet, dass die Zubereitung zusätzlich mindestens ein Silicon vom Typ der Dimethicone Copolyole enthält. Bevorzugte Silicone vom Typ der Dimethicone Copolyole sind dabei Silicone der Formel (Si-1),

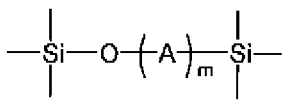

(Si-1),

mit

A = 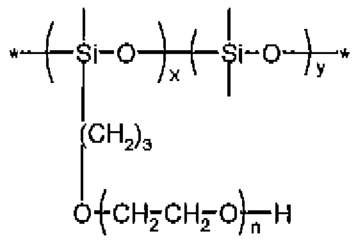

,

worin

- m für eine ganze Zahl von 1 bis 1000 steht,
- x und y jeweils unabhängig voneinander für eine Zahl von 1 bis 500 stehen, wobei, falls m ≥ 2 ist, die jeweiligen Werte x und y in einem Strukturelement A jeweils unabhängig von vorangehenden Strukturelementen A gewählt werden können,
- n für eine Zahl von 1 bis 50, bevorzugt 3 bis 30, weiter bevorzugt 8 bis 20, besonders bevorzugt 10 bis 15 steht.

Beispiele für bevorzugte und besonders geeignete hydrophil modifizierte Silicone sind die unter den INCI-Bezeichnungen PEG-12 Dimethicone (Handelsprodukt Dow Corning 193 C Fluid), PEG-17 Dimethicone (Handelsprodukt Silsoft 895 dimethicone copolyol), PEG-8 Dimethicone (Biowax liquid 754), PEG-10 Dimethicone (Handelsprodukte Dow Corning ES 5612 oder Silsoft 860). PEG-14 Dimethicone (Handelsprodukt Abil B 8843) bekannten Verbindungen.

In einer besonderen Ausführungsform des ersten Erfindungsgegenstands sind erfindungsgemäße Zubereitungen daher dadurch gekennzeichnet, dass das oder die Silicone vom Typ der Dimethicone Copolyole in einem Anteil von 0,05 bis 5,0 Gew.-%, bevorzugt von 0,1 bis 4,0 Gew.-%, insbesondere von 0,2 bis 3,0 Gew.-% und besonders bevorzugt von 0,3 bis 2,0 Gew.-%, jeweils bezogen auf das Gesamtgewicht der Zubereitung, enthalten ist.

Ferner können die erfindungsgemäßen Zubereitungen weitere Wirk-, Hilfs- und Zusatzstoffe enthalten, insbesondere weitere Tenside. Tenside sind im Sinne der vorliegenden Erfindung grenzflächenaktive Substanzen, insbesondere anionische, amphotere, zwitterionische, weitere nichtionische und kationische Tenside gemäß nachstehender Definitionen.

Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe

mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen);

Ethercarbonsäuren, insbesondere der Formel $RO(CH_2CH_2O)_xCH_2COOH$, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare $\alpha$-Olefinsulfonate; Sulfonate ungesättigter Fettsäuren; $\alpha$-Sulfofettsäuremethylester von Fettsäuren; Alkylsulfate und Alkylethersulfate, insbesondere der Formel $RO(CH_2CH_2O)_xSO_3H$, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel $RO(C_2H_4O)_xP(=O)(OH)(OR')$, worin R für einen aliphatischen, gegebenenfalls ungesättigten Kohlenwasserstoffrest mit 8 bis 30 Kohlenstoffatomen, R' für Wasserstoff, einen Rest $(CH_2CH_2O)_yR$ und x und y unabhängig voneinander für eine Zahl von 1 bis 10 steht; sulfatierte Fettsäurealkylenglykolester der Formel $RC(O)O(alkO)_nSO_3H$, in der R für einen linearen oder verzweigten, aliphatischen, gesättigten und/oder ungesättigten Alkylrest mit 6 bis 22 C-Atomen, alk für $CH_2CH_2$, $CHCH_3CH_2$ und/oder $CH_2CHCH_3$ und n für eine Zahl von 0,5 bis 5 steht; sowie Monoglyceridsulfate und Monoglyceridethersulfate. Erfindungsgemäß bevorzugte anionische Tenside sind Seifen, Alkylsulfate, Alkylethersulfate und Ethercarbonsäuren.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Beispiele solcher zwitterionischen Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer $C_8$-$C_{24}$-Alkyl-oder-Acylgruppe im Molekül mindestens eine freie Aminogruppe und mindestens eine -COOH- oder -$SO_3H$-Gruppe enthalten und zur Ausbildung innerer Salze befähigt sind. Übliche amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Beispielhafte amphotere Tenside sind N-Kokosalkylaminopropionat, Kokosacylaminoethylaminopröpionat, $C_{12}$-$C_{16}$-Acylsarcosin und insbesondere das unter der INCI-Bezeichnung bekannte Disodium Cocoamphodipropionate.

Weitere nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise neben den bereits beschriebenen nichtionischen Tensiden die Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder $C_2$-$C_6$-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol LS, Dehydol LT (BASF) erhältlichen Typen; Polyglycerinester und alkoxylierte Polyglycerinester, wie beispielsweise Poly(3)glycerin-diisostearat (Handelsprodukt: Lameform TGI (Henkel)) und Poly(2)glycerinpolyhydroxy-stearat (Handelsprodukt: Dehymuls PGPH (Henkel)); Polyolfettsäureester, wie beispielsweise das Handelsprodukt Hydagen HSP (BASF) oder Sovermol-Typen (BASF); höher alkoxylierte, propoxylierte und insbesondere ethoxylierte, Mono-, Diglyceride mit Alkoxylierungsgrad von größer als 5, wie beispielsweise Glycerinmonolaurat + 20 Ethylenoxid und Glycerinmonostearat + 20 Ethylenoxid; Aminoxide; Hydroxymischether; Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO); Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen In der Alkylkette und 5 bis 30 Ethylenoxid- und/oder Propylenoxid-Einheiten.

Zu den nichtionischen Emulgatoren im Sinne der Erfindung zählen weiterhin die Polymerisationsprodukte aus Ethylenoxid und Propylenoxid an gesättigte oder ungesättigte Fettsäureester mehrwertiger Alkohole mit gesättigten oder ungesättigten Fettsäuren; Alkylester von gesättigten oder ungesättigten Fettsäuren oder Alkylphenole und deren Alkoxylate; insbesondere Ethylenglykolether von Fettalkoholen; gemischte Ethylen- und Propylenglykolether mit Fettalkoholen; Fettsäureester an Sorbitan sowie Polyethylenglykol; Ester von nicht hydroxylierten $C_8$-$C_{30}$-Alkylmonocar-

bonäuren mit Polyethylenglykol; und Anlagerungsprodukte von Alkylphenolen an Ethylen- und/oder Propylenoxid. Erfindungsgemäße kationische Tenside sind monomere quartäre Ammoniumverbindungen, Esterquats und Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride sowie die unter den INCI-Bezeichnungen Quaternium-27 und Quaternium-83 bekannten Imidazolium-Verbindungen. Weitere erfindungsgemäße kationische Tenside stellen die quaternisierten Proteinhydrolysate dar. Alkylamidoamine werden üblicherweise durch Amidierung natürlicher oder synthetischer Fettsäuren und Fettsäureschnitte mit Dialkylaminoaminen hergestellt, wie Stearamidopropyl-dimethylamin. Ebenfalls bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex, Dehyquart und Armocare vertrieben. Die Produkte Armocare VGH-70, ein N,N-bis(2-Palmitoyloxyethyl)dimethylammoniumchlorid, sowie Dehyquart F-75, Dehyquart C-4046, Dehyquart L80 und Dehyquart AU-35 sind Beispiele für solche Esterquats.

Ferner können die erfindungsgemäßen Zubereitungen weitere Wirk-, Hilfs- und Zusatzstoffe enthalten, beispielsweise nichtionische Polymere, wie Vinylpyrrolidinon/Vinylacrylat-Copolymere, Polyvinylpyrrolidinon, Vinylpyrrolidinon/Vinylacetat-Copolymere, Polyethylenglykole und Polysiloxane; Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; anionische Polymere, wie Acrylsäure-Homo- und Copolymere, Xanthan, Natrium Carboxymethylcellulose; kationische oder amphotere Polymerverbindungen sind ausgewählt aus Polyquaternium-2, Polyquaternium-6, Polyquaternium-7, Polyquaternium-10, Polyquaternium-11, Polyquaternium-16, Polyquaternium-22, Polyquaternium-28, Polyquaternium-32, Polyquaternium-37, Polyquaternium-39, Polyquaternium-44, Polyquaternium-46, Polyquaternium-55 und/oder Polyquaternium-68; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Aminosäuren und Oligopeptide , insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate; pflanzliche Öle, wie Macadamianussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl; Lichtschutzmittel; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, $B_3$, $B_5$, $B_6$, C, E, F und H; Pflanzenextrakte; Fette und Wachse wie Fettalkohole, Bienenwachs, Montanwachs und Paraffine; Quell- und Penetrationsstoffe wie Glycerin, Propylenglykolmonoethylether, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat sowie Pigmente.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Es hat sich gezeigt, dass die Zubereitungen in Anwesenheit von Treibmitteln, insbesondere Treibgasen, besonders stabile, cremige, langlebige Schäume ausbilden.

Eine weitere Ausführungsform des ersten Erfindungsgegenstand ist daher dadurch gekennzeichnet, dass die Zubereitung zusätzlich mindestens ein Treibmittel enthält.

Als Treibmittel lassen sich alle in der Kosmetik gängigen Treibmittel einsetzen. Bevorzugt sind dabei Treibgase, wie Chlorfluorkohlenstoffe (CFK) bzw. Chlorfluorkohlenwasserstoffe (FCKW), komprimierter Stickstoff, $N_2O$, $CO_2$, Dimethylether, Propan und Butan, insbesondere kurzkettige Alkane wie Propan, Butan oder deren Gemische.

Der Anteil der Treibmittel in den erfindungsgemäßen Zubereitungen hängt von der gewünschten Schaummenge ab. Er beträgt zumeist zwischen 1 bis 10 Gew.-%, bevorzugt 3 bis 8 Gew.-%, bezogen auf das Gesamtgewicht der Zubereitung.

Im Falle des Zusatzes von Treibgas liegt die Zubereitung in Form eines Schaums vor. Erfindungsgemäß ist ein Schaum als Gas-Flüssigkeits-Gemisch definiert. Ein Schaum kennzeichnet dabei Gebilde aus gasgefüllten, kugel- oder polyederförmigen Zellen (Poren), welche durch flüssige, halbflüssige oder hochviskose Zellstege begrenzt werden.

Erfindungsgemäß bevorzugt enthält der Schaum als Gas die Treibgase.

Der Gasanteil des Schaums beträgt vorzugsweise mindestens 50 Vol-%, bevorzugt mindestens 70 Vol-% und weiter bevorzugt mindestens 80 Vol-%, jeweils bezogen auf das Gesamtvolumen des anwendungsbereiten Mittels,

Wenn die Volumenkonzentration des den Schaum bildenden Gases bei homodisperser Verteilung kleiner als 74% ist, so sind die Gasblasen wegen der oberflächenverkleinernden Wirkung der Grenzflächenspannung kugelförmig. Oberhalb der Grenze der dichtesten Kugelpackung werden die Blasen zu polyedrischen Lamellen deformiert, die von ca. 4 bis 600 nm dünnen Häutchen begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum.

Erfindungsgemäß besonders geeignete Schäume weisen ein Gas-Flüssigkeits-Verhältnis von 5 bis 50 mL/g, bevorzugt 10 bis 40 mL/g und insbesondere 15 bis 35 mL/g auf. Ein solches Gas-Flüssigkeits-Verhältnis wird dabei beispielsweise dadurch bestimmt, dass nach abgeschlossenem Mischvorgang bei Raumtemperatur und einer Ruhezeit von 1 min das Volumen des Schaums, beispielsweise mittels Messzylinder, bei bekanntem Gewicht der Zubereitung bestimmt wird. Alternativ kann auch ein bestimmtes Volumen, beispielsweise durch Abgabe in einen Messzylinder entnommen werden und dessen Gewicht durch Auswaage bestimmt werden.

Weiterhin hat es sich als besonders bevorzugt erwiesen, wenn die Farbveränderungszubereitung dünnflüssig formuliert ist. Zusammengeführte Mischung, die eine Viskosität von 0 bis 2000 mPa·s aufweisen (gemessen bei 22°C im Brookfield-Viskosimeter Typ RV-T mit Spindel LV-1 beziehungsweise RV-1 und einer Geschwindigkeit von 30U/min), haben sich als besonders bevorzugt erwiesen. Eine Viskosität von 0 bis 1000 mPa·s, gemessen unter den genannten Bedingungen, ist erfindungsgemäß besonders bevorzugt. Eine Viskosität von 5 bis 500 mPa·s, insbesondere von 10 bis 50 mPa·s (jeweils gemessen untern den oben genannten Bedingungen) ist erfindungsgemäß ganz besonders bevorzugt.

Die erfindungsgemäße Zubereitung kann weiterhin zur Farbveränderung bevorzugt zusätzlich als Oxidationsmittel Wasserstoffperoxid enthalten. Erfindungsgemäß ist darunter Wasserstoffperoxid oder dessen Anlagerungsprodukte an Harnstoff, Melamin sowie Natriumborat in Frage. Besonders bevorzugt enthält die Zubereitung Wasserstoffperoxid als Oxidationsmittel selbst.

Bevorzugt enthält das anwendungsbereite Mittel das Oxidationsmittel, insbesondere Wasserstoffperoxid, in einem Anteil von 0,5 bis 18 Gew.-%, insbesondere 1 bis 15 Gew.-% und besonders bevorzugt von 2 bis 12 Gew.-%, jeweils bezogen auf das Gesamtgewicht der Zubereitung.

Wasserstoffperoxid und farbverändernde Mittel werden aus Stabilitätsgründen erst unmittelbar vor der Anwendung oder während der Anwendung miteinander in Kontakt gebracht. Dazu kann es bevorzugt sein, die Zubereitung des ersten Erfindungsgegenstands und die Oxidationsmittelzubereitung, enthaltend Wasserstoffperoxid, getrennt voneinander bereitzustellen und erst kurz vor oder während der Anwendung mit der Zubereitung des ersten Erfindungsgegenstands zu vermischen.

Zur Stabilisierung von Wasserstoffperoxid während der Lagerung ist es insbesondere bevorzugt, wenn diese Oxidationsmittelzubereitung vor dem Vermischen einen sauren pH-Wert aufweist, insbesondere zwischen 2,5 und 5,5, bevorzugt zwischen 3,0 und 5,0. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Zur Stabilisierung von Wasserstoffperoxid in der Oxidationsmittelzubereitung ist es weiterhin bevorzugt, so genannte Komplexbildner einzusetzen. Komplexbildner sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt. Die Zahl der gebundenen Liganden hängt von der Koordinationszahl des zentralen Ions ab. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz. Auch Dipicolinsäure wird erfindungsgemäß vorzugsweise als Komplexbildner eingesetzt. Mittel, die eine Kombination aus einem EDTA-Salz, HEDP und Dipicolinsäure enthalten sind erfindungsgemäß besonders bevorzugt. Die anwendungsbereite Zubereitung besitzt einen alkalischen pH-Wert, bevorzugt zwischen 7 uns 12, bevorzugt zwischen 8 und 11,5 und insbesondere zwischen 8,5 und 11,0. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig.

Die erfindungsgemäße Farbveranderungszubereitung enthält daher bevorzugt zusätzlich mindestens ein Alkalisierungsmittel. Die Alkalisierungsmittel der Alkalisierungszubereitung werden typischerweise gewählt aus anorganischen Salzen, insbesondere der Alkali- und Erdalkalimetalle, organischen Alkalisierungsmitteln, insbesondere Aminen,

basische Aminosäuren und Alkanolaminen, und Ammoniak.

Erfindungsgemäß einsetzbare, organische Alkalisierungsmittel werden bevorzugt ausgewählt aus Alkanolaminen. Alkanolamine sind primäre, sekundäre oder tertiäre Amine mit einem $C_2$-$C_6$-Alkylgrundkörper, der mindestens eine Hydroxylgruppe trägt. Erfindungsgemäß bevorzugte Alkanolamine werden ausgewählt aus Triethanolamin, 2-Aminoethan-1-ol (Monoethanolamin), 2-Amino-2-methylpropan-1-ol und 2-Amino-2-methyl-propan-1,3-diol. Ein besonders bevorzugtes Alkanolamin ist Monoethanolamin. Geeignete basische Aminsäuren sind beispielsweise Lysin, Arginin und Ornithin. Erfindungsgemäße, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Ammoniumcarbonat, Natriumcarbonat und Kaliumcarbonat.

Das Alkalisierungsmittel liegt dabei in ausreichender Menge in der erfindungsgemäßen Farbveränderungszubereitung vor, um dem anwendungsbereiten Mittel nach Vermischen mit der Oxidationsmittelzubereitung einen alkalischen pH-Wert, bevorzugt von 8 bis 12, zu verleihen. Bevorzugt enthalten erfindungsgemäße Farbveränderungszubereitungen das oder die Alkalisierungsmittel in einem Anteil von 1 bis 25 Gew.-%, insbesondere 2 bis 15 Gew.-%, bevorzugt 2,5 bis 10 Gew.-%, jeweils bezogen auf das Gesamtgewicht der Farbveränderungszubereitung.

Wenn die Zubereitung des ersten Erfindungsgegenstands ein Treibmittel, insbesondere ein Treibgas, enthält, wird sie bevorzugt in einem druckresistenten Behältnis aufbewahrt. Es ist aus Gründen der Verpackungstechnik dann vorteilhaft, die Oxidationsmittelzubereitung gleichzeitig mit der Zubereitung des ersten Erfindungsgegenstands aufzubringen. Dazu kann es vorteilhaft sein, wenn die Oxidationsmittelzubereitung gleichfalls mindestens ein Treibmittel, bevorzugt ein Treibgas, wie voranstehend spezifiziert, enthält. Dadurch liegt auch die Oxidationsmittelzubereitung bei der Ausbringung als Schaum, bevorzugten mit den voranstehend für Schaum genannten Spezifikationen, vor.

Die Zubereitung gemäß erstem Erfindungsgegenstand, enthaltend zusätzlich mindestens ein Treibmittel, und die Oxidationsmittelzubereitung, enthaltend mindestens ein Treibmittel, werden dabei entweder aus zwei verschiedenen Behältern gleichzeitig aufgetragen oder bevorzugt aus einem Doppelkammerbehälter, der über zwei druckresistente Vorratsbehältnisse mit einem gemeinsamen oder zwei getrennten Auslässen verfügt, ausgebracht. Bevorzugt lassen sich im Falle von zwei Auslässen beide über ein gemeinsames Bedienelement öffnen und schließen.

Ein weiterer Gegenstand der vorliegenden Erfindung ist daher ein Verfahren zur Farbveränderung keratinischer Fasern, welches dadurch gekennzeichnet ist, dass

(i) eine Zubereitung (A) gemäß dem ersten Erfindungsgegenstand, enthaltend zusätzlich mindestens ein Treibmittel, und eine Oxidationsmittelzubereitung (B), enthaltend in einem kosmetischen Träger mindestens ein Oxidationsmittel und ein Treibmittel, gemeinsam und gleichzeitig auf die Fasern ausgetragen werden,
(ii) Zubereitung (A) und Oxidationsmittelzubereitung (B) auf den Fasern innig miteinander vermischt werden und gleichmäßig auf den Fasern verteilt werden,
(iii) und nach einer Einwirkzeit von 10 bis 45 min bei einer Temperatur von 20 bis 45°C aus den Fasern ausgespült werden.

Im ersten Verfahrensschritt (i) werden die beiden Zubereitungen (A) und (B) gemeinsam und gleichzeitig auf den keratinischen Fasern ausgebracht. Dazu liegen die Zubereitungen (A) und (B) in getrennt verpackten, vorzugsweise druckresistenten Vorratsbehältnissen vor. Die Behältnisse verfügen über ein manuelles, vorzugsweise mit einem Finger betätigbares Ventil als Auslass für die jeweilige Zubereitung. Bevorzugt werden Doppelkammerbehälter eingesetzt, deren beiden Behälter jeweils über ein Auslassventil verfügen. Mittels Auslassventil kann der Behälter geöffnet oder verschlossen werden. Besonders bevorzugt werden die beiden Auslassventile über ein gemeinsames Bedienelement gesteuert, so dass entweder beide Ventile geöffnet oder beide Ventile geschlossen sind. Dadurch wird eine gleichzeitige, gemeinsame Austragung beider Mittel auf die Fasern gewährleistet. Durch Dimensionsanpassung der Ventile und Druckanpassung in den beiden Vorratsbehältnissen kann dabei die Menge der beiden Zubereitungen nach Bedarf aufeinander abgestimmt werden. Die Zubereitungen (A) und (B) werden aus den Auslassventilen aus den Behältern ausgetragen.

Dabei können die Zubereitungen entweder unvermischt und nebeneinander durch zwei verschiedene Auslasskanäle oder in einem gemeinsamen Auslasskanal zumindest teilweise miteinander vermischt abgegeben werden. Die Auslasskanäle können über zusätzliche mechanische Hilfsmittel zur Einstellung der Schaumeigenschaften, wie Netzen mit bestimmten Porengrößen, verfügen.

Dem Fachmann sind solche Doppelkammerbehälter wohlbekannt, wie sie beispielsweise von der Firma TOYO Aerosol Industry Co. Ltd. bezogen werden können.

Im zweiten Verfahrensschritt (ii) werden die beiden Zubereitungen (A) und (B) durch manuelles Verkneten der Zubereitungen miteinander vermischt und die resultierende Mischung gleichmäßig auf den Fasern verteilt. Bei einem Verfahren zur Farbveränderung menschlicher Haare kann das anwendungsbereite Mittel dabei direkt auf dem Kopfhaar des Anwenders ausgebracht, vermischt und verteilt werden. Bevorzugt erfolgt auch die Verteilung manuell, gegebenenfalls auch mit weiteren Hilfsmitteln, wie Kamm oder Bürste. Bevorzugt wird der direkte Kontakt zwischen anwendungsbereiten Mittel und Hände durch die Verwendung von geeigneten Handschuhen, wie Einweghandschuhen, beispielsweise aus Latex, vermieden.

Im dritten Verfahrensschritt (iii) verbleibt das anwendungsbereite Mittel für einen Zeitraum von 10 bis 45 min auf den zu behandelnden Fasern. Bevorzugt liegt der Zeitraum zwischen 20 bis 40 min.

Die Anwendungstemperaturen können in einem Bereich zwischen 20 und 45°C liegen. Gegebenenfalls kann während der Verbleibzeit des Mittels auf den Fasern auch durch externe Wärmequellen eine höhere oder genau definierte Temperatur eingestellt werden. Besonders bevorzugt ist es, die Farbveränderung durch physikalische Maßnahmen zu unterstützen. Erfindungsgemäße Verfahren, bei denen die Anwendung durch Einwirkung von Wärme, IR- und/oder UV-Strahlung während der Einwirkzeit unterstützt wird, können dabei bevorzugt sein.

Nach der Einwirkungszeit gemäß dritten Verfahrensschritts werden die verbliebenen Anteile der Zubereitungen durch Ausspülen von den zu behandelnden Fasern entfernt. Hierzu werden die Fasern mit Wasser und/oder einer wässrigen Tensid-Zubereitung ausgespült. Üblicherweise wird hierzu 20 °C bis 40 °C warmes Wasser bzw. entsprechend temperierte wässrige Tensid-Zubereitung eingesetzt.

Zur Anwendung des erfindungsgemäßen Verfahrens werden die Zubereitungen und Behälter bevorzugt in einer Verpackungseinheit angeboten.

Ein dritter Gegenstand der vorliegenden Erfindung ist daher eine Mehrkomponentenverpackungseinheit (Kit-of-Parts), welche

(a) mindestens einen Container (I), der eine Zubereitung (A) gemäß dem ersten Erfindungsgegenstand, enthaltend zusätzlich mindestens ein Treibmittel, beinhaltet, und
(b) mindestens einen Container (II), der eine Oxidationsmittelzubereitung (B), enthaltend in einem kosmetischen Träger mindestens ein Oxidationsmittel und ein Treibmittel, beinhaltet,

umfasst.

Die voneinander getrennt konfektionierten Zubereitungen werden dazu in physikalisch getrennten Containern bereitgestellt. Der Begriff "Container" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff "Container" umfasst daher - ohne darauf beschränkt zu sein, das Innere einer Tube, eines Beutels oder Sackes, eines Kanisters, einer Dose, einer Wanne, einer Flasche, eines Glases oder eines Paketes, eines Kartons, einer Box, eines Umschlages oder eines anderen Behältnisses. Die Komponenten der Zubereitungen können in einem einzelnen Container, der mehrere Kompartimente zur Aufnahme der Zubereitungen aufweist, enthalten sein, es ist aber auch möglich und gegebenenfalls bevorzugt, sie auf verschiedene Container aufzuteilen.

Besonders bevorzugt ist es dabei, wenn die Container (I) und (II) jeweils ein Vorratsbehältnis eines voranstehend beschriebenen Doppelkammerbehälters darstellen, bei dem sich die Auslassventile der Vorratsbehältnisse über ein gemeinsames Bedienelement steuern lassen.

Eine bevorzugte Ausführungsform des dritten Erfindungsgegenstands ist eine Mehrkomponentenverpackungseinheit, bei der die Container (I) und (II) jeweils als Auslass ein Ventil aufweisen, welche dadurch gekennzeichnet ist, dass die Ventile der beiden Container (I) und (II) sich gleichzeitig manuell mittels eines gemeinsamen Bedienelements öffnen und schließen lassen.

Das gemeinsame Bedienelement ist vorzugsweise eine tasten- oder druckknopf-artige Einheit, die bei ausgeübtem Druck, insbesondere mit einem Finger, die beiden Ventile der Container (I) und (II) gleichzeitig öffnet und nach Beendigung des ausgeübten Drucks diese wieder gleichzeitig schließt. Bezüglich weiterer bevorzugter Ausführungsformen des erfindungsgemäßen Kits gilt mutatis mutandis das zu den erfindungsgemäßen Verfahren und Zubereitungen Gesagte.

Beispiele

Es wurden die folgenden Zubereitungen hergestellt. Die Mengenangaben verstehen sich, soweit nichts anderes vermerkt ist, jeweils in Gewichtsprozent.

*1 Farbveränderungszuhereitungen*

Tabelle 1a

| **Rohstoffe** | **V1** | **V2** | **E1** | **E2** |
|---|---|---|---|---|
| Plantacare 818 UP | 5,00 | 5,00 | 5,00 | 5,00 |

(fortgesetzt)

| Rohstoffe | V1 | V2 | E1 | E2 |
|---|---|---|---|---|
| Dimethylcocoylbetain | 5,00 | 5,00 | 5,00 | - |
| Cremophor CO 60 | 2,00 | 2,00 | 2,00 | - |
| Kaliumolelat | - | 2,60 | 2,60 | 3,75 |
| Comperlan 100 | - | - | 1,50 | 2,00 |
| Dow Corning 193 C Fluid | - | - | - | 0,50 |
| Ethanol, 96% | 5,00 | 5,00 | 5,00 | 0,50 |
| EDTA | 0,20 | 0,20 | 0,20 | - |
| p-Toluylendiaminsulfat | 1,35 | 1,35 | 1,35 | 1,35 |
| 4-Chlorresorcin | 0,06 | 0,06 | 0,06 | 0,06 |
| 3-Aminophenol | 0,02 | 0,02 | 0,02 | 0,02 |
| 5-Amino-2-methylphenol | 0,72 | 0,72 | 0,72 | 0,72 |
| Ascorbinsäure | 0,05 | 0,05 | 0,05 | - |
| Natriumsulfit, wasserfrei | 0,20 | 0,20 | 0,20 | - |
| L-Serin | 1,00 | 1,00 | 1,00 | - |
| Merquat 281 | 3,00 | 3,00 | 3,00 | - |
| Monoethanolamin | 6,00 | 6,00 | 6,00 | 4,00 |
| Parfum | qs | | | |
| Wasser | ad 100 | | | |

Tabelle 1b

| Rohstoffe | E5 |
|---|---|
| Kaliumoleat, 12,5% | 16,0 |
| Plantacare 818 UP | 10,00 |
| Cremophor CO 60 | 4,00 |
| Comperlan 100 | 1,50 |
| Dow Corning 193 C Fluid | 1,00 |
| Cetrimonium Bromide | - |
| Phospholipid EFA | - |
| Ethanol, 96% | 5,00 |
| HEDP, 60% | 0,20 |
| Kaliumhydroxid, 50% | 0,50 |
| p-Toluylendiaminsulfat | 1,80 |
| 1-(2-Hydroxyethyl)-4,5-diamino-pyrazol sulfat | 0,50 |
| 2-Methylresorcin | 0,45 |
| 3-Aminophenol | 0,18 |
| 4-Chlorresorcin | 0,02 |
| 2-Amino-3-hydroxypyridin | 0,40 |

(fortgesetzt)

| **Rohstoffe** | **E5** |
|---|---|
| Resorcin | 0,02 |
| 5-Amino-2-methylphenol | 0,25 |
| 2-Amino-6-chloro-4-nitrophenol | 0,20 |
| HC Blue No. 12 | 0,03 |
| Ascorbinsäure | 0,05 |
| Natriumsulfit | 0,20 |
| L-Serin | 1,00 |
| Monoethanolamin | 6,00 |
| Parfum | qs |
| Wasser | ad 100 |

*2 Oxidationsmittelzubereitung*

Tabelle 2

| Rohstoff | OX-1 |
|---|---|
| Natronlauge, 45%. | 0,73 |
| Dipicolinsäure | 0,1 |
| Dinatriumpyrophosphat | 0,03 |
| HEDP, 60% | 1,5 |
| Plantacare 818 UP | 5,0 |
| Cremophor CO 60 | 2,0 |
| Eumulgin L | 0,4 |
| Wasserstoffperoxid (wässrig, 50 %) | 15,0 |
| Wasser | ad 100 |

*3 Verzeichnis der eingesetzten Handelsprodukte*

| | |
|---|---|
| Cremophor CO60 | INCI-Bezeichnung; PEG-60 Hydrogenated Castor Oil (BASF) |
| Comperlan 100 | ca. 92-99% Aktivsubstanzgehalt; INCI-Bezeichnung: Cocamide MEA (BASF) |
| Plantacare 818 UP | ca. 51-53% Aktivsubstanzgehalt; INCI-Bezeichnung: Coco-Glucoside, Aqua (Water) (BASF) |
| Dow Corning 193 C Fluid | INCI-Bezeichnung: PEG-12 Dimethicone (Dow Corning) |
| Merquat 281 | ca. 39-43% Aktivsubstanzgehalt; INCI-Bezeichnung: Polyquaternium-22, Aqua (Water) (Nalco) |
| Eumulgin L | INCI-Bezeichnung: PPG-1-PEG-9 Lauryl Glycol Ether (BASF) |
| Phospholipid EFA | ca. 30% Aktivsubstanz; INCI-Bezeichnung: Linoleamidopropyl PGdimonium Chloride Phosphate, water (45%), propylene glycol (25%) (Uniquema) |

*4 Ausfärbungen*

Die Farbveränderungszubereitungen V1, V2, E1 bis E4 und die Oxidationsmittelzubereitung OX-1 wurden jeweils im Verhältnis 95 : 5 mit einem Treibgasgemisch aus Propan/Butan (1:1) in einem druckresistenten Behälter versetzt.

Die Farbveränderungszubereitungen V1 und V2 sowie E1 bis E4 wurden jeweils mit der Oxidationsmittelzubereitung OX-1 aus den Vorratsbehältern ("Container") eines Doppelkammerbehälters mit gemeinsamen Bedienelement der Auslassventile der Vorratsbehälter auf Strähnen von Haaren (Büffelbauchhaar/BB bzw. Euronaturhaar/ENH, Kerling, weiß) im Verhältnis 1 : 1 aufgebracht, und die Zubereitungen wurden durch inniges Verkneten auf den Fasern miteinander vermischt. Die Anwendungsmischung verblieb für eine Einwirkdauer von 30 min bei Raumtemperatur auf den Haaren. Anschließend wurde das verbliebene Mittel aus den Haaren mit lauwarmen Wasser ca. 2 min lang ausgespült und das Haar wurde mit einem Handtuch getrocknet.

Die farbmetrischen Messungen erfolgten auf der Strähne an jeweils 4 Messpunkten. Als Messgerät diente der Spectralflash SF 450 der Firma Datacolor.

Die Ergebnisse der Messungen wurden mithilfe des CIELAB Farbenraums quantifiziert.

Der L-Wert steht dabei für die Helligkeit der Färbung (schwarz-weiß-Achse); je größer der Wert für L ist, desto heller ist die Färbung. Der a-Wert steht für die rot-grün-Achse des Systems; je größer der Wert ist, umso mehr ist die Färbung ins Rote verschoben. Der b-Wert steht für die gelb-blau-Achse des Systems; je größer der Wert ist, umso mehr ist die Färbung ins Gelbe verschoben. Farbunterschiede werden mittels des ΔE-Wertes charakterisiert:

$$\Delta E=\sqrt{(L_1^*-L_2^*)^2+(a_1^*-a_2^*)^2+(b_1^*-b_2^*)^2}$$

Folgende Färbeergebnisse wurden erhalten (Tabelle 3):

| Haartyp | Rezeptur | L* | a* | b* | ΔE |
|---|---|---|---|---|---|
| BB | unbehandelt | 72,98 | 2,27 | 22,57 | |
| BB | V1 | 30,37 | 14,02 | -3,04 | 51,1 |
| BB | V2 | 24,83 | 13,42 | -2,76 | 55,5 |
| BB | E1 | 22,69 | 12,78 | -3,92 | 57,8 |
| BB | E2 | 22,61 | 13,32 | -4,09 | 58,1 |
| EHN | unbehandelt | 72,68 | -0,23 | 8,78 | |
| EHN | V1 | 25,88 | 15,29 | -7,06 | 51,8 |
| EHN | V2 | 25,68 | 14,62 | -6,71 | 51,7 |
| EHN | E1 | 21,42 | 13,86 | -6,34 | 55,3 |
| EHN | E2 | 19,57 | 12,48 | -5,67 | 56,5 |

Die Verwendung der erfindungsgemäßen Färbezubereitungen E1 und E2 führte zu einer deutlichen Verbesserung der Farbintensität (ΔE-Wert) der Färbung auf beiden Haartypen gegenüber den nicht erfindungsgemäßen Zubereitungen V1 (ohne Seife und ohne nichtionisches Tensid gemäß Formel (I)) und V2 (ohne nichtionisches Tensid gemäß Formel (I)).

Es wurden gleichmäßige, haltbare und glänzende Färbungen von hoher Farbintensität und Farbigkeit erhalten.

**Patentansprüche**

**1.** Zubereitung zur oxidativen Farbveränderung keratinischer Fasern, enthaltend in einem kosmetisch verträglichen Träger,

(a) mindestens ein farbveränderndes Mittel und
(b) die Seife Kaliumoleat in einem Anteil von 1,5 bis 4,0 Gew.-%, bezogen auf das Gesamtgewicht der Zubereitung,
(c) zusätzlich Cocamide MEA als nichtionisches Tensid in einem Anteil von 0,6 bis 2,5 Gew.-%, bezogen auf das Gesamtgewicht der Zubereitung,

wobei Kaliumoleat und Cocamide MEA in einem Gewichtsverhältnis von 4,0 / 1 bis 1,2 / 1 enthalten sind, **dadurch gekennzeichnet, dass** die Zubereitung zusätzlich mindestens ein weiteres nichtionisches Tensid, ausgewählt aus Alkylpolyglucosiden und/oder alkoxylierten Fettsäureestern, enthält.

2. Zubereitung nach Anspruch 1, **dadurch gekennzeichnet, dass** die Zubereitung zusätzlich mindestens ein Treibmittel enthält.

3. Verfahren zur oxidativen Farbveränderung keratinischer Fasern, **dadurch gekennzeichnet, dass**

(i) eine Zubereitung (A) gemäß Anspruch 2 und eine Oxidationsmittelzubereitung (B), enthaltend in einem kosmetischen Träger mindestens ein Oxidationsmittel und ein Treibmittel, gemeinsam und gleichzeitig auf die Fasern ausgetragen werden,
(ii) Zubereitung (A) und Oxidationsmittelzubereitung (B) gleichmäßig auf den Fasern verteilt werden,
(iii) und nach einer Einwirkzeit von 10 bis 45 min bei einer Temperatur von 20 bis 45°C aus den Fasern ausgespült werden.

4. Mehrkomponentenverpackungseinheit (Kit-of-Parts), umfassend

a) mindestens einen Container (I), der eine Zubereitung (A) gemäß Anspruch 2 enthält, und
b) mindestens einen Container (II), der eine Oxidationsmittelzubereitung (B), enthaltend in einem kosmetischen Träger mindestens ein Oxidationsmittel und ein Treibmittel, enthält.

5. Mehrkomponentenverpackungseinheit nach Anspruch 4, bei der die Container (I) und (II) jeweils als Auslass ein Ventil aufweisen, **dadurch gekennzeichnet, dass** die Ventile der beiden Container (I) und (II) sich gleichzeitig manuell mittels eines gemeinsamen Bedienelements öffnen und schließen lassen.

## Claims

1. A preparation for oxidatively changing the color of keratin fibers, containing, in a cosmetically acceptable carrier,

(a) at least one color-changing agent and
(b) the soap potassium oleate in a proportion of from 1.5 to 4.0 wt.%, based on the total weight of the preparation,
(c) additionally cocamide MEA as a non-ionic surfactant in a proportion of from 0.6 to 2.5 wt.%, based on the total weight of the preparation,

potassium oleate and cocamide MEA being contained in a weight ratio of from 4.0/1 to 1.2/1,
**characterized in that** the preparation additionally contains at least one additional non-ionic surfactant, selected from alkyl polyglucosides and/or alkoxylated fatty acid esters.

2. The preparation according to claim 1, **characterized in that** the preparation additionally contains at least one propellant.

3. A method for oxidatively changing the color of keratin fibers, **characterized in that**

(i) a preparation (A) according to claim 2 and an oxidizing agent preparation (B), containing, in a cosmetic carrier, at least one oxidizing agent and a propellant, are applied to the fibers together and at the same time,
(ii) preparation (A) and oxidizing agent preparation (B) are distributed uniformly onto the fibers,
(iii) and are rinsed out of the fibers after a contact time of from 10 to 45 minutes at a temperature of from 20 to 45 °C.

4. A multi-component packaging unit (kit of parts), comprising

a) at least one container (I), which contains a preparation (A) according to claim 2, and
b) at least one container (II), which contains an oxidizing agent preparation (B), containing, in a cosmetic carrier,

at least one oxidizing agent and a propellant.

**5.** The multi-component packaging unit according to claim 4, in which the containers (I) and (II) each comprise a valve as an outlet, **characterized in that** the valves of the two containers (I) and (II) can be simultaneously manually opened and closed by means of a common control element.

**Revendications**

**1.** Préparation destinée à la modification, par oxydation, de la couleur de fibres kératiniques, contenant, dans un support cosmétiquement acceptable,

a) au moins un agent modificateur de couleur, et
b) du savon d'oléate de potassium dans une proportion de 1,5 à 4,0 % en poids, par rapport au poids total de la préparation,
c) en outre du cocamide MEA comme tensioactif non ionique dans une proportion de 0,6 à 2,5 % en poids, par rapport au poids total de la préparation,

dans laquelle l'oléate de potassium et le cocamide MEA sont présents dans un rapport pondéral de 4,0/1 à 1,2/1, **caractérisée en ce que** la préparation contient en outre au moins un autre agent tensioactif non ionique choisi parmi les alkylpolyglucosides et/ou les esters d'acides gras alcoxylés.

**2.** Préparation selon la revendication 1, **caractérisée en ce que** la préparation contient en outre au moins un agent propulseur.

**3.** Procédé pour la modification, par oxydation, de la couleur de fibres kératiniques, **caractérisé**

i) **en ce qu'**une préparation (A) selon la revendication 2 et une préparation d'agent oxydant (B) contenant, dans un support cosmétique, au moins un agent oxydant et un agent propulseur, sont conjointement et simultanément appliquées sur les fibres,
ii) **en ce que** la préparation (A) et la préparation d'agent oxydant (B) sont réparties de manière uniforme sur les fibres,
iii) et **en ce que** les fibres sont rincées après un temps de pose de 10 à 45 min à une température de 20 à 45 °C.

**4.** Unité de conditionnement à composants multiples (kit), comprenant

a) au moins un conteneur (I) contenant une préparation (A) selon la revendication 2, et
b) au moins un conteneur (II) qui contient une préparation d'agent oxydant (B) contenant au moins un agent oxydant et un agent propulseur dans un support cosmétique.

**5.** Unité de conditionnement à composants multiples selon la revendication 4, dans laquelle les conteneurs (I) et (II) comportent chacun une soupape comme sortie, **caractérisée en ce que** les soupapes des deux conteneurs (I) et (II) s'ouvrent et se ferment manuellement, simultanément au moyen d'un élément de commande commun.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2277600 A1


### In der Beschreibung aufgeführte Nicht-Patentliteratur


- Grundlagen und Rezepturen der Kosmetika. **KH. SCHRADER.** Handbücher. Hüthig Buch Verlag, 1989